# EUROPEAN PATENT SPECIFICATION

(11) **EP 2 673 378 B1**
(45) Date of publication and mention of the grant of the patent: **10.12.2014**
(21) Application number: 12703530.1
(22) Date of filing: 09.02.2012
(51) Int. Cl.: C12Q 1/68

(54) **METHOD FOR LABELLING DOUBLE-STRANDED DNA OR DNA/RNA HYBRIDS**
VERFAHREN ZUM MARKIEREN VON DOPPELSTRÄNGIGER DNA ODER DNA/RNA-HYBRIDEN
PROCÉDÉ POUR MARQUER DE L'ADN BICATÉNAIRE OU DES HYBRIDES ADN/ARN

(30) Priority: 09.02.2011 EP 11153889
(43) Date of publication of application: 18.12.2013
(73) Proprietor: RiboxX GmbH, 01445 Radebeul (DE)
(72) Inventor: ROHAYEM, Jacques, 01277 Dresden (DE)
(74) Representative: Habermann, Hruschka & Schnabel
(86) International application number: PCT/EP2012/052247
(87) International publication number: WO 2012/107538

(56) References cited:
- WO-A1-2012/038448
- WO-A1-2012/038450
- US-A1- 2008 176 293
- ROHAYEM JACQUES ET AL: "Characterization of norovirus 3Dpol RNA-dependent RNA polymerase activity and initiation of RNA synthesis", JOURNAL OF GENERAL VIROLOGY, SOCIETY FOR GENERAL MICROBIOLOGY, SPENCERS WOOD, GB, vol. 87, no. Pt 9, 1 September 2006 (2006-09-01), pages 2621-2630, XP002544506, ISSN: 0022-1317, DOI: 10.1099/VIR.0.81802-0
- SIEGEL R W ET AL: "Use of DNA, RNA, and chimeric templates by a viral RNA-dependent RNA polymerase: evolutionary implications for the transition from the RNA to the DNA world", JOURNAL OF VIROLOGY, THE AMERICAN SOCIETY FOR MICROBIOLOGY, US, vol. 73, no. 8, 1 August 1999 (1999-08-01) , pages 6424-6429, XP002665261, ISSN: 0022-538X

### SEQUENCE LISTING

<110> RiboxX GmbH
<120> Method for labelling double-stranded DNA or DNA/RNA hybrids
<130> R 0088 WO
<150> EP 11153889.8
   <151> 2011-02-09
<160> 18
<170> PatentIn version 3.3
<210> 1
   <211> 5
   <212> PRT
   <213> Artificial
<220>
   <223> A-motif
<220>
   <221> misc_feature
   <222> (1)..(2)
   <223> Xaa = any amino acid
<400> 1
   Xaa Xaa Asp Tyr Ser
   1               5
<210> 2
   <211> 5
   <212> PRT
   <213> Artificial
<220>
   <223> B-motif
<220>
   <221> misc_feature
   <222> (2)..(2)
   <223> Xaa = any amino acid
<400> 2
   Gly Xaa Pro Ser Gly
   1               5
<210> 3
   <211> 4
   <212> PRT
   <213> Artificial
<220>
   <223> C-motif
<400> 3
   Tyr Gly Asp Asp
   1
<210> 4
   <211> 5
   <212> PRT
   <213> Artificial
<220>
   <223> D-motif
<220>
   <221> misc_feature
   <222> (1)..(2)
   <223> Xaa = any amino acid
<400> 4
   Xaa Xaa Tyr Gly Leu
   1 5
<210> 5
   <211> 10
   <212> PRT
   <213> Artificial
<220>
   <223> E-Motif
<220>
   <221> misc_feature
   <222> (1)..(4)
   <223> Xaa = any amino acid
<220>
   <221> misc_feature
   <222> (7)..(7)
   <223> Xaa = any amino acid
<220>
   <221> misc_feature
   <222> (9)..(10)
   <223> Xaa = any amino acid
<400> 5
   Xaa Xaa Xaa Xaa Phe Leu Xaa Arg Xaa Xaa
   1 5 10
<210> 6
   <211> 520
   <212> PRT
   <213> Norovirus
<400> 6
   Met Gly Gly Asp Ser Lys Gly Thr Tyr Cys Gly Ala Pro Ile Leu Gly
   1 5 10 15
   Pro Gly Ser Ala Pro Lys Leu Ser Thr Lys Thr Lys Phe Trp Arg Ser
   20 25 30
   Ser Thr Thr Pro Leu Pro Pro Gly Thr Tyr Glu Pro Ala Tyr Leu Gly
   35 40 45
   Gly Lys Asp Pro Arg Val Lys Gly Gly Pro Ser Leu Gln Gln Val Met
   50 55 60
   Arg Asp Gln Leu Lys Pro Phe Thr Glu Pro Arg Gly Lys Pro Pro Lys
   65 70 75 80
   Pro Ser Val Leu Glu Ala Ala Lys Lys Thr Ile Ile Asn Val Leu Glu
   85 90 95
   Gln Thr Ile Asp Pro Pro Glu Lys Trp Ser Phe Thr Gln Ala Cys Ala
   100 105 110
   Ser Leu Asp Lys Thr Thr Ser Ser Gly His Pro His His Met Arg Lys
   115 120 125
   Asn Asp Cys Trp Asn Gly Glu Ser Phe Thr Gly Lys Leu Ala Asp Gln
   130 135 140
   Ala Ser Lys Ala Asn Leu Met Phe Glu Gly Gly Lys Asn Met Thr Pro
   145 150 155 160
   Val Tyr Thr Gly Ala Leu Lys Asp Glu Leu Val Lys Thr Asp Lys Ile
   165 170 175
   Tyr Gly Lys Ile Lys Lys Arg Leu Leu Trp Gly Ser Asp Leu Ala Thr
   180 185 190
   Met Ile Arg Cys Ala Arg Ala Phe Gly Gly Leu Met Asp Glu Leu Lys
   195 200 205
   Ala His Cys Val Thr Leu Pro Ile Arg Val Gly Met Asn Met Asn Glu
   210 215 220
   Asp Gly Pro Ile Ile Phe Glu Arg His Ser Arg Tyr Lys Tyr His Tyr
   225 230 235 240
   Asp Ala Asp Tyr Ser Arg Trp Asp Ser Thr Gln Gln Arg Ala Val Leu
   245 250 255
   Ala Ala Ala Leu Glu Ile Met Val Lys Phe Ser Ser Glu Pro His Leu
               260                 265                 270
   Ala Gln Val Val Ala Glu Asp Leu Leu Ser Pro Ser Val Val Asp Val
           275                 280                 285
   Gly Asp Phe Lys Ile Ser Ile Asn Glu Gly Leu Pro Ser Gly Val Pro
       290                 295                 300
   Cys Thr Ser Gln Trp Asn Ser Ile Ala His Trp Leu Leu Thr Leu Cys
   305                 310                 315                 320
   Ala Leu Ser Glu Val Thr Asn Leu Ser Pro Asp Ile Ile Gln Ala Asn
                   325                 330                 335
   Ser Leu Phe Ser Phe Tyr Gly Asp Asp Glu Ile Val Ser Thr Asp Ile
               340                 345                 350
   Lys Leu Asp Pro Glu Lys Leu Thr Ala Lys Leu Lys Glu Tyr Gly Leu
           355                 360                 365
   Lys Pro Thr Arg Pro Asp Lys Thr Glu Gly Pro Leu Val Ile Ser Glu
       370                 375                 380
   Asp Leu Asn Gly Leu Thr Phe Leu Arg Arg Thr Val Thr Arg Asp Pro
   385                 390                 395                 400
   Ala Gly Trp Phe Gly Lys Leu Glu Gln Ser Ser Ile Leu Arg Gln Met
                   405                 410                 415
   Tyr Trp Thr Arg Gly Pro Asn His Glu Asp Pro Ser Glu Thr Met Ile
               420                 425                 430
   Pro His Ser Gln Arg Pro Ile Gln Leu Met Ser Leu Leu Gly Glu Ala
           435                 440                 445
   Ala Leu His Gly Pro Ala Phe Tyr Ser Lys Ile Ser Lys Leu Val Ile
       450                 455                 460
   Ala Glu Leu Lys Glu Gly Gly Met Asp Phe Tyr Val Pro Arg Gln Glu
   465                 470                 475                 480
   Pro Met Phe Arg Trp Met Arg Phe Ser Asp Leu Ser Thr Trp Glu Gly
                   485                 490                 495
   Asp Arg Asn Leu Ala Pro Ser Phe Val Asn Glu Asp Gly Val Glu Val
               500                 505                 510
   Asp Lys Leu Ala Ala Ala Leu Glu
           515                 520
<210> 7
   <211> 517
   <212> PRT
   <213> Sapovirus
<400> 7
   Met Lys Asp Glu Phe Gln Trp Lys Gly Leu Pro Val Val Lys Ser Gly
   1               5                   10                  15
   Leu Asp Val Gly Gly Met Pro Thr Gly Thr Arg Tyr His Arg Ser Pro
               20                  25                  30
   Ala Trp Pro Glu Glu Gln Pro Gly Glu Thr His Ala Pro Ala Pro Phe
           35                  40                  45
   Gly Ala Gly Asp Lys Arg Tyr Thr Phe Ser Gln Thr Glu Met Leu Val
       50                  55                  60
   Asn Gly Leu Lys Pro Tyr Thr Glu Pro Thr Ala Gly Val Pro Pro Gln
   65                  70                  75                  80
   Leu Leu Ser Arg Ala Val Thr His Val Arg Ser Tyr Ile Glu Thr Ile
                   85                  90                  95
   Ile Gly Thr His Arg Ser Pro Val Leu Thr Tyr His Gln Ala Cys Glu
               100                 105                 110
   Leu Leu Glu Arg Thr Thr Ser Cys Gly Pro Phe Val Gln Gly Leu Lys
           115                 120                 125
   Gly Asp Tyr Trp Asp Glu Glu Gln Gln Gln Tyr Thr Gly Val Leu Ala
       130                 135                 140
   Asn His Leu Glu Gln Ala Trp Asp Lys Ala Asn Lys Gly Ile Ala Pro
   145                 150                 155                 160
   Arg Asn Ala Tyr Lys Leu Ala Leu Lys Asp Glu Leu Arg Pro Ile Glu
                   165                 170                 175
   Lys Asn Lys Ala Gly Lys Arg Arg Leu Leu Trp Gly Cys Asp Ala Ala
               180                 185                 190
   Thr Thr Leu Ile Ala Thr Ala Ala Phe Lys Ala Val Ala Thr Arg Leu
           195                 200                 205
   Gln Val Val Thr Pro Met Thr Pro Val Ala Val Gly Ile Asn Met Asp
   210 215 220
   Ser Val Gln Met Gln Val Met Asn Asp Ser Leu Lys Gly Gly Val Leu
   225 230 235 240
   Tyr Cys Leu Asp Tyr Ser Lys Trp Asp Ser Thr Gln Asn Pro Ala Val
   245 250 255
   Thr Ala Ala Ser Leu Ala Ile Leu Glu Arg Phe Ala Glu Pro His Pro
   260 265 270
   Ile Val Ser Cys Ala Ile Glu Ala Leu Ser Ser Pro Ala Glu Gly Tyr
   275 280 285
   Val Asn Asp Ile Lys Phe Val Thr Arg Gly Gly Leu Pro Ser Gly Met
   290 295 300
   Pro Phe Thr Ser Val Val Asn Ser Ile Asn His Met Ile Tyr Val Ala
   305 310 315 320
   Ala Ala Ile Leu Gln Ala Tyr Glu Ser His Asn Val Pro Tyr Thr Gly
   325 330 335
   Asn Val Phe Gln Val Glu Thr Val His Thr Tyr Gly Asp Asp Cys Met
   340 345 350
   Tyr Ser Val Cys Pro Ala Thr Ala Ser Ile Phe His Ala Val Leu Ala
   355 360 365
   Asn Leu Thr Ser Tyr Gly Leu Lys Pro Thr Ala Ala Asp Lys Ser Asp
   370 375 380
   Ala Ile Lys Pro Thr Asn Thr Pro Val Phe Leu Lys Arg Thr Phe Thr
   385 390 395 400
   Gln Thr Pro His Gly Val Arg Ala Leu Leu Asp Ile Thr Ser Ile Thr
   405 410 415
   Arg Gln Phe Tyr Trp Leu Lys Ala Asn Arg Thr Ser Asp Pro Ser Ser
   420 425 430
   Pro Pro Ala Phe Asp Arg Gln Ala Arg Ser Ala Gln Leu Glu Asn Ala
   435 440 445
   Leu Ala Tyr Ala Ser Gln His Gly Pro Val Val Phe Asp Thr Val Arg
       450                 455                 460
   Gln Ile Ala Ile Lys Thr Ala Gln Gly Glu Gly Leu Val Leu Val Asn
   465                 470                 475                 480
   Thr Asn Tyr Asp Gln Ala Leu Ala Thr Tyr Asn Ala Trp Phe Ile Gly
                   485                 490                 495
   Gly Thr Val Pro Asp Pro Val Gly His Thr Glu Gly Thr His Lys Ile
               500                 505                 510
   Val Phe Glu Met Glu
           515
<210> 8
   <211> 533
   <212> PRT
   <213> Vesivirus
<400> 8
   Met Lys Val Thr Thr Gln Lys Tyr Asp Val Thr Lys Pro Asp Ile Ser
   1               5                   10                  15
   Tyr Lys Gly Leu Ile Cys Lys Gln Leu Asp Glu Ile Arg Val Ile Pro
               20                  25                  30
   Lys Gly Thr Arg Leu His Val Ser Pro Ala His Thr Asp Asp Tyr Asp
           35                  40                  45
   Glu Cys Ser His Gln Pro Ala Ser Leu Gly Ser Gly Asp Pro Arg Cys
       50                  55                  60
   Pro Lys Ser Leu Thr Ala Ile Val Val Asp Ser Leu Lys Pro Tyr Cys
   65                  70                  75                  80
   Glu Lys Thr Asp Gly Pro Pro His Asp Ile Leu His Arg Val Gln Arg
                   85                  90                  95
   Met Leu Ile Asp His Leu Ser Gly Phe Val Pro Met Asn Ile Ser Ser
               100                 105                 110
   Glu Pro Ser Met Leu Ala Ala Phe His Lys Leu Asn His Asp Thr Ser
           115                 120                 125
   Cys Gly Pro Tyr Leu Gly Gly Arg Lys Lys Asp His Met Ile Gly Gly
       130                 135                 140
   Glu Pro Asp Lys Pro Leu Leu Asp Leu Leu Ser Ser Lys Trp Lys Leu
   145 150 155 160
   Ala Thr Gln Gly Ile Gly Leu Pro His Glu Tyr Thr Ile Gly Leu Lys
   165 170 175
   Asp Glu Leu Arg Pro Val Glu Lys Val Gln Glu Gly Lys Arg Arg Met
   180 185 190
   Ile Trp Gly Cys Asp Val Gly Val Ala Thr Val Cys Ala Ala Ala Phe
   195 200 205
   Lys Gly Val Ser Asp Ala Ile Thr Ala Asn His Gln Tyr Gly Pro Val
   210 215 220
   Gln Val Gly Ile Asn Met Asp Gly Pro Ser Val Glu Ala Leu Tyr Gln
   225 230 235 240
   Arg Ile Arg Ser Ala Ala Lys Val Phe Ala Val Asp Tyr Ser Lys Trp
   245 250 255
   Asp Ser Thr Gln Ser Pro Arg Val Ser Ala Ala Ser Ile Asp Ile Leu
   260 265 270
   Arg Tyr Phe Ser Asp Arg Ser Pro Ile Val Asp Ser Ala Ala Asn Thr
   275 280 285
   Leu Lys Ser Pro Pro Ile Ala Ile Phe Asn Gly Val Ala Val Lys Val
   290 295 300
   Thr Ser Gly Leu Pro Ser Gly Met Pro Leu Thr Ser Val Ile Asn Ser
   305 310 315 320
   Leu Asn His Cys Leu Tyr Val Gly Cys Ala Ile Leu Gln Ser Leu Glu
   325 330 335
   Ser Arg Asn Ile Pro Val Thr Trp Asn Leu Phe Ser Thr Phe Asp Met
   340 345 350
   Met Thr Tyr Gly Asp Asp Gly Val Tyr Met Phe Pro Met Met Phe Ala
   355 360 365
   Ser Val Ser Asp Gln Ile Phe Ala Asn Leu Thr Ala Tyr Gly Leu Lys
   370 375 380
   Pro Thr Arg Val Asp Lys Ser Val Gly Ala Ile Glu Pro Ile Asp Pro
   385 390 395 400
   Glu Ser Val Val Phe Leu Lys Arg Thr Ile Thr Arg Thr Pro His Gly
   405 410 415
   Ile Arg Gly Leu Leu Asp Arg Gly Ser Ile Ile Arg Gln Phe Tyr Tyr
   420 425 430
   Ile Lys Gly Glu Asn Ser Asp Asp Trp Lys Thr Pro Pro Lys Thr Ile
   435 440 445
   Asp Pro Thr Ser Arg Gly Gln Gln Leu Trp Asn Ala Cys Leu Tyr Ala
   450 455 460
   Ser Gln His Gly Pro Glu Phe Tyr Asn Lys Val Tyr Arg Leu Ala Glu
   465 470 475 480
   Lys Ala Val Glu Tyr Glu Glu Leu His Phe Glu Pro Pro Ser Tyr His
   485 490 495
   Ser Ala Leu Glu His Tyr Asn Asn Gln Phe Asn Gly Val Asp Thr Arg
   500 505 510
   Ser Asp Gln Ile Asp Ala Ser Val Met Thr Asp Leu His Cys Asp Val
   515 520 525
   Phe Glu Val Leu Glu
   530
<210> 9
   <211> 517
   <212> PRT
   <213> Lagovirus
<400> 9
   Met Thr Ser Asn Phe Phe Cys Gly Glu Pro Ile Asp Tyr Arg Gly Ile
   1 5 10 15
   Thr Ala His Arg Leu Val Gly Ala Glu Pro Arg Pro Pro Val Ser Gly
   20 25 30
   Thr Arg Tyr Ala Lys Val Pro Gly Val Pro Asp Glu Tyr Lys Thr Gly
   35 40 45
   Tyr Arg Pro Ala Asn Leu Gly Arg Ser Asp Pro Asp Ser Asp Lys Ser
   50 55 60
   Leu Met Asn Ile Ala Val Lys Asn Leu Gln Val Tyr Gln Gln Glu Pro
   65 70 75 80
   Lys Leu Asp Lys Val Asp Glu Phe Ile Glu Arg Ala Ala Ala Asp Val
                   85                  90                  95
   Leu Gly Tyr Leu Arg Phe Leu Thr Lys Gly Glu Arg Gln Ala Asn Leu
               100                 105                 110
   Asn Phe Lys Ala Ala Phe Asn Thr Leu Asp Leu Ser Thr Ser Cys Gly
           115                 120                 125
   Pro Phe Val Pro Gly Lys Lys Ile Asp His Val Lys Asp Gly Val Met
       130                 135                 140
   Asp Gln Val Leu Ala Lys His Leu Tyr Lys Cys Trp Ser Val Ala Asn
   145                 150                 155                 160
   Ser Gly Lys Ala Leu His His Ile Tyr Ala Cys Gly Leu Lys Asp Glu
                   165                 170                 175
   Leu Arg Pro Leu Asp Lys Val Lys Glu Gly Lys Lys Arg Leu Leu Trp
               180                 185                 190
   Gly Cys Asp Val Gly Val Ala Val Cys Ala Ala Ala Val Phe His Asn
           195                 200                 205
   Ile Cys Tyr Lys Leu Lys Met Val Ala Arg Phe Gly Pro Ile Ala Val
       210                 215                 220
   Gly Val Asp Met Thr Ser Arg Asp Val Asp Val Ile Ile Asn Asn Leu
   225                 230                 235                 240
   Thr Ser Lys Ala Ser Asp Phe Leu Cys Leu Asp Tyr Ser Lys Trp Asp
                   245                 250                 255
   Ser Thr Met Ser Pro Cys Val Val Arg Leu Ala Ile Asp Ile Leu Ala
               260                 265                 270
   Asp Cys Cys Glu Gln Thr Glu Leu Thr Lys Ser Val Val Leu Thr Leu
           275                 280                 285
   Lys Ser His Pro Met Thr Ile Leu Asp Ala Met Ile Val Gln Thr Lys
       290                 295                 300
   Arg Gly Leu Pro Ser Gly Met Pro Phe Thr Ser Val Ile Asn Ser Ile
   305                 310                 315                 320
   Cys His Trp Leu Leu Trp Ser Ala Ala Val Tyr Lys Ser Cys Ala Glu
                   325                 330                 335
   Ile Gly Leu His Cys Ser Asn Leu Tyr Glu Asp Ala Pro Phe Tyr Thr
   340 345 350
   Tyr Gly Asp Asp Gly Val Tyr Ala Met Thr Pro Met Met Val Ser Leu
   355 360 365
   Leu Pro Ala Ile Ile Glu Asn Leu Arg Asp Tyr Gly Leu Ser Pro Thr
   370 375 380
   Ala Ala Asp Lys Thr Glu Phe Ile Asp Val Cys Pro Leu Asn Lys Ile
   385 390 395 400
   Ser Phe Leu Lys Arg Thr Phe Glu Leu Thr Asp Ile Gly Trp Val Ser
   405 410 415
   Lys Leu Asp Lys Ser Ser Ile Leu Arg Gln Leu Glu Trp Ser Lys Thr
   420 425 430
   Thr Ser Arg His Met Val Ile Glu Glu Thr Tyr Asp Leu Ala Lys Glu
   435 440 445
   Glu Arg Gly Val Gln Leu Glu Glu Leu Gln Val Ala Ala Ala Ala His
   450 455 460
   Gly Gln Glu Phe Phe Asn Phe Val Cys Arg Glu Leu Glu Arg Gln Gln
   465 470 475 480
   Ala Tyr Thr Gln Phe Ser Val Tyr Ser Tyr Asp Ala Ala Arg Lys Ile
   485 490 495
   Leu Ala Asp Arg Lys Arg Val Val Ser Val Val Pro Asp Asp Glu Phe
   500 505 510
   Val Asn Val Met Glu
   515
<210> 10
   <211> 526
   <212> PRT
   <213> Artificial
<220>
   <223> Norovirus RdRp having His tag
<400> 10
   Met Gly Gly Asp Ser Lys Gly Thr Tyr Cys Gly Ala Pro Ile Leu Gly
   1 5 10 15
   Pro Gly Ser Ala Pro Lys Leu Ser Thr Lys Thr Lys Phe Trp Arg Ser
   20 25 30
   Ser Thr Thr Pro Leu Pro Pro Gly Thr Tyr Glu Pro Ala Tyr Leu Gly
   35 40 45
   Gly Lys Asp Pro Arg Val Lys Gly Gly Pro Ser Leu Gln Gln Val Met
   50 55 60
   Arg Asp Gln Leu Lys Pro Phe Thr Glu Pro Arg Gly Lys Pro Pro Lys
   65 70 75 80
   Pro Ser Val Leu Glu Ala Ala Lys Lys Thr Ile Ile Asn Val Leu Glu
   85 90 95
   Gln Thr Ile Asp Pro Pro Glu Lys Trp Ser Phe Thr Gln Ala Cys Ala
   100 105 110
   Ser Leu Asp Lys Thr Thr Ser Ser Gly His Pro His His Met Arg Lys
   115 120 125
   Asn Asp Cys Trp Asn Gly Glu Ser Phe Thr Gly Lys Leu Ala Asp Gln
   130 135 140
   Ala Ser Lys Ala Asn Leu Met Phe Glu Gly Gly Lys Asn Met Thr Pro
   145 150 155 160
   Val Tyr Thr Gly Ala Leu Lys Asp Glu Leu Val Lys Thr Asp Lys Ile
   165 170 175
   Tyr Gly Lys Ile Lys Lys Arg Leu Leu Trp Gly Ser Asp Leu Ala Thr
   180 185 190
   Met Ile Arg Cys Ala Arg Ala Phe Gly Gly Leu Met Asp Glu Leu Lys
   195 200 205
   Ala His Cys Val Thr Leu Pro Ile Arg Val Gly Met Asn Met Asn Glu
   210 215 220
   Asp Gly Pro Ile Ile Phe Glu Arg His Ser Arg Tyr Lys Tyr His Tyr
   225 230 235 240
   Asp Ala Asp Tyr Ser Arg Trp Asp Ser Thr Gln Gln Arg Ala Val Leu
   245 250 255
   Ala Ala Ala Leu Glu Ile Met Val Lys Phe Ser Ser Glu Pro His Leu
   260 265 270
   Ala Gln Val Val Ala Glu Asp Leu Leu Ser Pro Ser Val Val Asp Val
           275                 280                 285
   Gly Asp Phe Lys Ile Ser Ile Asn Glu Gly Leu Pro Ser Gly Val Pro
       290                 295                 300
   Cys Thr Ser Gln Trp Asn Ser Ile Ala His Trp Leu Leu Thr Leu Cys
   305                 310                 315                 320
   Ala Leu Ser Glu Val Thr Asn Leu Ser Pro Asp Ile Ile Gln Ala Asn
                   325                 330                 335
   Ser Leu Phe Ser Phe Tyr Gly Asp Asp Glu Ile Val Ser Thr Asp Ile
               340                 345                 350
   Lys Leu Asp Pro Glu Lys Leu Thr Ala Lys Leu Lys Glu Tyr Gly Leu
           355                 360                 365
   Lys Pro Thr Arg Pro Asp Lys Thr Glu Gly Pro Leu Val Ile Ser Glu
       370                 375                 380
   Asp Leu Asn Gly Leu Thr Phe Leu Arg Arg Thr Val Thr Arg Asp Pro
   385                 390                 395                 400
   Ala Gly Trp Phe Gly Lys Leu Glu Gln Ser Ser Ile Leu Arg Gln Met
                   405                 410                 415
   Tyr Trp Thr Arg Gly Pro Asn His Glu Asp Pro Ser Glu Thr Met Ile
               420                 425                 430
   Pro His Ser Gln Arg Pro Ile Gln Leu Met Ser Leu Leu Gly Glu Ala
           435                 440                 445
   Ala Leu His Gly Pro Ala Phe Tyr Ser Lys Ile Ser Lys Leu Val Ile
       450                 455                 460
   Ala Glu Leu Lys Glu Gly Gly Met Asp Phe Tyr Val Pro Arg Gln Glu
   465                 470                 475                 480
   Pro Met Phe Arg Trp Met Arg Phe Ser Asp Leu Ser Thr Trp Glu Gly
                   485                 490                 495
   Asp Arg Asn Leu Ala Pro Ser Phe Val Asn Glu Asp Gly Val Glu Val
               500                 505                 510
   Asp Lys Leu Ala Ala Ala Leu Glu His His His His His His
           515                 520                 525
<210> 11
   <211> 526
   <212> PRT
   <213> Artificial
<220>
   <223> Norovirus RdRp having His tag
<400> 11
   Met His His His His His His Gly Gly Asp Ser Lys Gly Thr Tyr Cys
   1               5                   10                  15
   Gly Ala Pro Ile Leu Gly Pro Gly Ser Ala Pro Lys Leu Ser Thr Lys
               20                  25                  30
   Thr Lys Phe Trp Arg Ser Ser Thr Thr Pro Leu Pro Pro Gly Thr Tyr
           35                  40                  45
   Glu Pro Ala Tyr Leu Gly Gly Lys Asp Pro Arg Val Lys Gly Gly Pro
       50                  55                  60
   Ser Leu Gln Gln Val Met Arg Asp Gln Leu Lys Pro Phe Thr Glu Pro
   65                  70                  75                  80
   Arg Gly Lys Pro Pro Lys Pro Ser Val Leu Glu Ala Ala Lys Lys Thr
                   85                  90                  95
   Ile Ile Asn Val Leu Glu Gln Thr Ile Asp Pro Pro Glu Lys Trp Ser
               100                 105                 110
   Phe Thr Gln Ala Cys Ala Ser Leu Asp Lys Thr Thr Ser Ser Gly His
           115                 120                 125
   Pro His His Met Arg Lys Asn Asp Cys Trp Asn Gly Glu Ser Phe Thr
       130                 135                 140
   Gly Lys Leu Ala Asp Gln Ala Ser Lys Ala Asn Leu Met Phe Glu Gly
   145                 150                 155                 160
   Gly Lys Asn Met Thr Pro Val Tyr Thr Gly Ala Leu Lys Asp Glu Leu
                   165                 170                 175
   Val Lys Thr Asp Lys Ile Tyr Gly Lys Ile Lys Lys Arg Leu Leu Trp
               180                 185                 190
   Gly Ser Asp Leu Ala Thr Met Ile Arg Cys Ala Arg Ala Phe Gly Gly
           195                 200                 205
   Leu Met Asp Glu Leu Lys Ala His Cys Val Thr Leu Pro Ile Arg Val
       210                 215                 220
   Gly Met Asn Met Asn Glu Asp Gly Pro Ile Ile Phe Glu Arg His Ser
   225                 230                 235                 240
   Arg Tyr Lys Tyr His Tyr Asp Ala Asp Tyr Ser Arg Trp Asp Ser Thr
                   245                 250                 255
   Gln Gln Arg Ala Val Leu Ala Ala Ala Leu Glu Ile Met Val Lys Phe
               260                 265                 270
   Ser Ser Glu Pro His Leu Ala Gln Val Val Ala Glu Asp Leu Leu Ser
           275                 280                 285
   Pro Ser Val Val Asp Val Gly Asp Phe Lys Ile Ser Ile Asn Glu Gly
       290                 295                 300
   Leu Pro Ser Gly Val Pro Cys Thr Ser Gln Trp Asn Ser Ile Ala His
   305                 310                 315                 320
   Trp Leu Leu Thr Leu Cys Ala Leu Ser Glu Val Thr Asn Leu Ser Pro
                   325                 330                 335
   Asp Ile Ile Gln Ala Asn Ser Leu Phe Ser Phe Tyr Gly Asp Asp Glu
               340                 345                 350
   Ile Val Ser Thr Asp Ile Lys Leu Asp Pro Glu Lys Leu Thr Ala Lys
           355                 360                 365
   Leu Lys Glu Tyr Gly Leu Lys Pro Thr Arg Pro Asp Lys Thr Glu Gly
       370                 375                 380
   Pro Leu Val Ile Ser Glu Asp Leu Asn Gly Leu Thr Phe Leu Arg Arg
   385                 390                 395                 400
   Thr Val Thr Arg Asp Pro Ala Gly Trp Phe Gly Lys Leu Glu Gln Ser
                   405                 410                 415
   Ser Ile Leu Arg Gln Met Tyr Trp Thr Arg Gly Pro Asn His Glu Asp
               420                 425                 430
   Pro Ser Glu Thr Met Ile Pro His Ser Gln Arg Pro Ile Gln Leu Met
           435                 440                 445
   Ser Leu Leu Gly Glu Ala Ala Leu His Gly Pro Ala Phe Tyr Ser Lys
       450                 455                 460
   Ile Ser Lys Leu Val Ile Ala Glu Leu Lys Glu Gly Gly Met Asp Phe
   465                 470                 475                 480
   Tyr Val Pro Arg Gln Glu Pro Met Phe Arg Trp Met Arg Phe Ser Asp
                   485                 490                 495
   Leu Ser Thr Trp Glu Gly Asp Arg Asn Leu Ala Pro Ser Phe Val Asn
               500                 505                 510
   Glu Asp Gly Val Glu Val Asp Lys Leu Ala Ala Ala Leu Glu
           515                 520                 525
<210> 12
   <211> 523
   <212> PRT
   <213> Artificial
<220>
   <223> Sapovirus RdRp having his tag
<400> 12
   Met Lys His His His His His His Asp Glu Phe Gln Trp Lys Gly Leu
   1               5                   10                  15
   Pro Val Val Lys Ser Gly Leu Asp Val Gly Gly Met Pro Thr Gly Thr
               20                  25                  30
   Arg Tyr His Arg Ser Pro Ala Trp Pro Glu Glu Gln Pro Gly Glu Thr
           35                  40                  45
   His Ala Pro Ala Pro Phe Gly Ala Gly Asp Lys Arg Tyr Thr Phe Ser
       50                  55                  60
   Gln Thr Glu Met Leu Val Asn Gly Leu Lys Pro Tyr Thr Glu Pro Thr
   65                  70                  75                  80
   Ala Gly Val Pro Pro Gln Leu Leu Ser Arg Ala Val Thr His Val Arg
                   85                  90                  95
   Ser Tyr Ile Glu Thr Ile Ile Gly Thr His Arg Ser Pro Val Leu Thr
               100                 105                 110
   Tyr His Gln Ala Cys Glu Leu Leu Glu Arg Thr Thr Ser Cys Gly Pro
           115                 120                 125
   Phe Val Gln Gly Leu Lys Gly Asp Tyr Trp Asp Glu Glu Gln Gln Gln
       130                 135                 140
   Tyr Thr Gly Val Leu Ala Asn His Leu Glu Gln Ala Trp Asp Lys Ala
   145                 150                 155                 160
   Asn Lys Gly Ile Ala Pro Arg Asn Ala Tyr Lys Leu Ala Leu Lys Asp
                   165                 170                 175
   Glu Leu Arg Pro Ile Glu Lys Asn Lys Ala Gly Lys Arg Arg Leu Leu
               180                 185                 190
   Trp Gly Cys Asp Ala Ala Thr Thr Leu Ile Ala Thr Ala Ala Phe Lys
           195                 200                 205
   Ala Val Ala Thr Arg Leu Gln Val Val Thr Pro Met Thr Pro Val Ala
       210                 215                 220
   Val Gly Ile Asn Met Asp Ser Val Gln Met Gln Val Met Asn Asp Ser
   225                 230                 235                 240
   Leu Lys Gly Gly Val Leu Tyr Cys Leu Asp Tyr Ser Lys Trp Asp Ser
                   245                 250                 255
   Thr Gln Asn Pro Ala Val Thr Ala Ala Ser Leu Ala Ile Leu Glu Arg
               260                 265                 270
   Phe Ala Glu Pro His Pro Ile Val Ser Cys Ala Ile Glu Ala Leu Ser
           275                 280                 285
   Ser Pro Ala Glu Gly Tyr Val Asn Asp Ile Lys Phe Val Thr Arg Gly
       290                 295                 300
   Gly Leu Pro Ser Gly Met Pro Phe Thr Ser Val Val Asn Ser Ile Asn
   305                 310                 315                 320
   His Met Ile Tyr Val Ala Ala Ala Ile Leu Gln Ala Tyr Glu Ser His
                   325                 330                 335
   Asn Val Pro Tyr Thr Gly Asn Val Phe Gln Val Glu Thr Val His Thr
               340                 345                 350
   Tyr Gly Asp Asp Cys Met Tyr Ser Val Cys Pro Ala Thr Ala Ser Ile
           355                 360                 365
   Phe His Ala Val Leu Ala Asn Leu Thr Ser Tyr Gly Leu Lys Pro Thr
       370                 375                 380
   Ala Ala Asp Lys Ser Asp Ala Ile Lys Pro Thr Asn Thr Pro Val Phe
   385                 390                 395                 400
   Leu Lys Arg Thr Phe Thr Gln Thr Pro His Gly Val Arg Ala Leu Leu
   405 410 415
   Asp Ile Thr Ser Ile Thr Arg Gln Phe Tyr Trp Leu Lys Ala Asn Arg
   420 425 430
   Thr Ser Asp Pro Ser Ser Pro Pro Ala Phe Asp Arg Gln Ala Arg Ser
   435 440 445
   Ala Gln Leu Glu Asn Ala Leu Ala Tyr Ala Ser Gln His Gly Pro Val
   450 455 460
   Val Phe Asp Thr Val Arg Gln Ile Ala Ile Lys Thr Ala Gln Gly Glu
   465 470 475 480
   Gly Leu Val Leu Val Asn Thr Asn Tyr Asp Gln Ala Leu Ala Thr Tyr
   485 490 495
   Asn Ala Trp Phe Ile Gly Gly Thr Val Pro Asp Pro Val Gly His Thr
   500 505 510
   Glu Gly Thr His Lys Ile Val Phe Glu Met Glu
   515 520
<210> 13
   <211> 523
   <212> PRT
   <213> Artificial
<220>
   <223> Saporvirus RdRp having His tag
<400> 13
   Met Lys Asp Glu Phe Gln Trp Lys Gly Leu Pro Val Val Lys Ser Gly
   1 5 10 15
   Leu Asp Val Gly Gly Met Pro Thr Gly Thr Arg Tyr His Arg Ser Pro
   20 25 30
   Ala Trp Pro Glu Glu Gln Pro Gly Glu Thr His Ala Pro Ala Pro Phe
   35 40 45
   Gly Ala Gly Asp Lys Arg Tyr Thr Phe Ser Gln Thr Glu Met Leu Val
   50 55 60
   Asn Gly Leu Lys Pro Tyr Thr Glu Pro Thr Ala Gly Val Pro Pro Gln
   65 70 75 80
   Leu Leu Ser Arg Ala Val Thr His Val Arg Ser Tyr Ile Glu Thr Ile
                   85                  90                  95
   Ile Gly Thr His Arg Ser Pro Val Leu Thr Tyr His Gln Ala Cys Glu
               100                 105                 110
   Leu Leu Glu Arg Thr Thr Ser Cys Gly Pro Phe Val Gln Gly Leu Lys
           115                 120                 125
   Gly Asp Tyr Trp Asp Glu Glu Gln Gln Gln Tyr Thr Gly Val Leu Ala
       130                 135                 140
   Asn His Leu Glu Gln Ala Trp Asp Lys Ala Asn Lys Gly Ile Ala Pro
   145                 150                 155                 160
   Arg Asn Ala Tyr Lys Leu Ala Leu Lys Asp Glu Leu Arg Pro Ile Glu
                   165                 170                 175
   Lys Asn Lys Ala Gly Lys Arg Arg Leu Leu Trp Gly Cys Asp Ala Ala
               180                 185                 190
   Thr Thr Leu Ile Ala Thr Ala Ala Phe Lys Ala Val Ala Thr Arg Leu
           195                 200                 205
   Gln Val Val Thr Pro Met Thr Pro Val Ala Val Gly Ile Asn Met Asp
       210                 215                 220
   Ser Val Gln Met Gln Val Met Asn Asp Ser Leu Lys Gly Gly Val Leu
   225                 230                 235                 240
   Tyr Cys Leu Asp Tyr Ser Lys Trp Asp Ser Thr Gln Asn Pro Ala Val
                   245                 250                 255
   Thr Ala Ala Ser Leu Ala Ile Leu Glu Arg Phe Ala Glu Pro His Pro
               260                 265                 270
   Ile Val Ser Cys Ala Ile Glu Ala Leu Ser Ser Pro Ala Glu Gly Tyr
           275                 280                 285
   Val Asn Asp Ile Lys Phe Val Thr Arg Gly Gly Leu Pro Ser Gly Met
       290                 295                 300
   Pro Phe Thr Ser Val Val Asn Ser Ile Asn His Met Ile Tyr Val Ala
   305                 310                 315                 320
   Ala Ala Ile Leu Gln Ala Tyr Glu Ser His Asn Val Pro Tyr Thr Gly
                   325                 330                 335
   Asn Val Phe Gln Val Glu Thr Val His Thr Tyr Gly Asp Asp Cys Met
               340                 345                 350
   Tyr Ser Val Cys Pro Ala Thr Ala Ser Ile Phe His Ala Val Leu Ala
           355                 360                 365
   Asn Leu Thr Ser Tyr Gly Leu Lys Pro Thr Ala Ala Asp Lys Ser Asp
       370                 375                 380
   Ala Ile Lys Pro Thr Asn Thr Pro Val Phe Leu Lys Arg Thr Phe Thr
   385                 390                 395                 400
   Gln Thr Pro His Gly Val Arg Ala Leu Leu Asp Ile Thr Ser Ile Thr
                   405                 410                 415
   Arg Gln Phe Tyr Trp Leu Lys Ala Asn Arg Thr Ser Asp Pro Ser Ser
               420                 425                 430
   Pro Pro Ala Phe Asp Arg Gln Ala Arg Ser Ala Gln Leu Glu Asn Ala
           435                 440                 445
   Leu Ala Tyr Ala Ser Gln His Gly Pro Val Val Phe Asp Thr Val Arg
       450                 455                 460
   Gln Ile Ala Ile Lys Thr Ala Gln Gly Glu Gly Leu Val Leu Val Asn
   465                 470                 475                 480
   Thr Asn Tyr Asp Gln Ala Leu Ala Thr Tyr Asn Ala Trp Phe Ile Gly
                   485                 490                 495
   Gly Thr Val Pro Asp Pro Val Gly His Thr Glu Gly Thr His Lys Ile
               500                 505                 510
   Val Phe Glu Met Glu His His His His His His
           515                 520
<210> 14
   <211> 539
   <212> PRT
   <213> Artificial
<220>
   <223> Vesivirus RdRp having His tag
<400> 14
   Met Lys Val Thr Thr Gln Lys Tyr Asp Val Thr Lys Pro Asp Ile Ser
   1               5                   10                  15
   Tyr Lys Gly Leu Ile Cys Lys Gln Leu Asp Glu Ile Arg Val Ile Pro
               20                  25                  30
   Lys Gly Thr Arg Leu His Val Ser Pro Ala His Thr Asp Asp Tyr Asp
           35                  40                  45
   Glu Cys Ser His Gln Pro Ala Ser Leu Gly Ser Gly Asp Pro Arg Cys
       50                  55                  60
   Pro Lys Ser Leu Thr Ala Ile Val Val Asp Ser Leu Lys Pro Tyr Cys
   65                  70                  75                  80
   Glu Lys Thr Asp Gly Pro Pro His Asp Ile Leu His Arg Val Gln Arg
                   85                  90                  95
   Met Leu Ile Asp His Leu Ser Gly Phe Val Pro Met Asn Ile Ser Ser
               100                 105                 110
   Glu Pro Ser Met Leu Ala Ala Phe His Lys Leu Asn His Asp Thr Ser
           115                 120                 125
   Cys Gly Pro Tyr Leu Gly Gly Arg Lys Lys Asp His Met Ile Gly Gly
       130                 135                 140
   Glu Pro Asp Lys Pro Leu Leu Asp Leu Leu Ser Ser Lys Trp Lys Leu
   145                 150                 155                 160
   Ala Thr Gln Gly Ile Gly Leu Pro His Glu Tyr Thr Ile Gly Leu Lys
                   165                 170                 175
   Asp Glu Leu Arg Pro Val Glu Lys Val Gln Glu Gly Lys Arg Arg Met
               180                 185                 190
   Ile Trp Gly Cys Asp Val Gly Val Ala Thr Val Cys Ala Ala Ala Phe
           195                 200                 205
   Lys Gly Val Ser Asp Ala Ile Thr Ala Asn His Gln Tyr Gly Pro Val
       210                 215                 220
   Gln Val Gly Ile Asn Met Asp Gly Pro Ser Val Glu Ala Leu Tyr Gln
   225                 230                 235                 240
   Arg Ile Arg Ser Ala Ala Lys Val Phe Ala Val Asp Tyr Ser Lys Trp
                   245                 250                 255
   Asp Ser Thr Gln Ser Pro Arg Val Ser Ala Ala Ser Ile Asp Ile Leu
               260                 265                 270
   Arg Tyr Phe Ser Asp Arg Ser Pro Ile Val Asp Ser Ala Ala Asn Thr
           275                 280                 285
   Leu Lys Ser Pro Pro Ile Ala Ile Phe Asn Gly Val Ala Val Lys Val
       290                 295                 300
   Thr Ser Gly Leu Pro Ser Gly Met Pro Leu Thr Ser Val Ile Asn Ser
   305                 310                 315                 320
   Leu Asn His Cys Leu Tyr Val Gly Cys Ala Ile Leu Gln Ser Leu Glu
                   325                 330                 335
   Ser Arg Asn Ile Pro Val Thr Trp Asn Leu Phe Ser Thr Phe Asp Met
               340                 345                 350
   Met Thr Tyr Gly Asp Asp Gly Val Tyr Met Phe Pro Met Met Phe Ala
           355                 360                 365
   Ser Val Ser Asp Gln Ile Phe Ala Asn Leu Thr Ala Tyr Gly Leu Lys
       370                 375                 380
   Pro Thr Arg Val Asp Lys Ser Val Gly Ala Ile Glu Pro Ile Asp Pro
   385                 390                 395                 400
   Glu Ser Val Val Phe Leu Lys Arg Thr Ile Thr Arg Thr Pro His Gly
                   405                 410                 415
   Ile Arg Gly Leu Leu Asp Arg Gly Ser Ile Ile Arg Gln Phe Tyr Tyr
               420                 425                 430
   Ile Lys Gly Glu Asn Ser Asp Asp Trp Lys Thr Pro Pro Lys Thr Ile
           435                 440                 445
   Asp Pro Thr Ser Arg Gly Gln Gln Leu Trp Asn Ala Cys Leu Tyr Ala
       450                 455                 460
   Ser Gln His Gly Pro Glu Phe Tyr Asn Lys Val Tyr Arg Leu Ala Glu
   465                 470                 475                 480
   Lys Ala Val Glu Tyr Glu Glu Leu His Phe Glu Pro Pro Ser Tyr His
                   485                 490                 495
   Ser Ala Leu Glu His Tyr Asn Asn Gln Phe Asn Gly Val Asp Thr Arg
               500                 505                 510
   Ser Asp Gln Ile Asp Ala Ser Val Met Thr Asp Leu His Cys Asp Val
   515 520 525
   Phe Glu Val Leu Glu His His His His His His
   530 535
<210> 15
   <211> 523
   <212> PRT
   <213> Artificial
<220>
   <223> Lagovirus RdRp having His tag
<400> 15
   Met Thr Ser Asn Phe Phe Cys Gly Glu Pro Ile Asp Tyr Arg Gly Ile
   1               5                   10                  15
   Thr Ala His Arg Leu Val Gly Ala Glu Pro Arg Pro Pro Val Ser Gly
               20                  25                  30
   Thr Arg Tyr Ala Lys Val Pro Gly Val Pro Asp Glu Tyr Lys Thr Gly
           35                  40                  45
   Tyr Arg Pro Ala Asn Leu Gly Arg Ser Asp Pro Asp Ser Asp Lys Ser
       50                  55                  60
   Leu Met Asn Ile Ala Val Lys Asn Leu Gln Val Tyr Gln Gln Glu Pro
   65                  70                  75                  80
   Lys Leu Asp Lys Val Asp Glu Phe Ile Glu Arg Ala Ala Ala Asp Val
                   85                  90                  95
   Leu Gly Tyr Leu Arg Phe Leu Thr Lys Gly Glu Arg Gln Ala Asn Leu
               100                 105                 110
   Asn Phe Lys Ala Ala Phe Asn Thr Leu Asp Leu Ser Thr Ser Cys Gly
           115                 120                 125
   Pro Phe Val Pro Gly Lys Lys Ile Asp His Val Lys Asp Gly Val Met
       130                 135                 140
   Asp Gln Val Leu Ala Lys His Leu Tyr Lys Cys Trp Ser Val Ala Asn
   145                 150                 155                 160
   Ser Gly Lys Ala Leu His His Ile Tyr Ala Cys Gly Leu Lys Asp Glu
                   165                 170                 175
   Leu Arg Pro Leu Asp Lys Val Lys Glu Gly Lys Lys Arg Leu Leu Trp
               180                 185                 190
   Gly Cys Asp Val Gly Val Ala Val Cys Ala Ala Ala Val Phe His Asn
           195                 200                 205
   Ile Cys Tyr Lys Leu Lys Met Val Ala Arg Phe Gly Pro Ile Ala Val
       210                 215                 220
   Gly Val Asp Met Thr Ser Arg Asp Val Asp Val Ile Ile Asn Asn Leu
   225                 230                 235                 240
   Thr Ser Lys Ala Ser Asp Phe Leu Cys Leu Asp Tyr Ser Lys Trp Asp
                   245                 250                 255
   Ser Thr Met Ser Pro Cys Val Val Arg Leu Ala Ile Asp Ile Leu Ala
               260                 265                 270
   Asp Cys Cys Glu Gln Thr Glu Leu Thr Lys Ser Val Val Leu Thr Leu
           275                 280                 285
   Lys Ser His Pro Met Thr Ile Leu Asp Ala Met Ile Val Gln Thr Lys
       290                 295                 300
   Arg Gly Leu Pro Ser Gly Met Pro Phe Thr Ser Val Ile Asn Ser Ile
   305                 310                 315                 320
   Cys His Trp Leu Leu Trp Ser Ala Ala Val Tyr Lys Ser Cys Ala Glu
                   325                 330                 335
   Ile Gly Leu His Cys Ser Asn Leu Tyr Glu Asp Ala Pro Phe Tyr Thr
               340                 345                 350
   Tyr Gly Asp Asp Gly Val Tyr Ala Met Thr Pro Met Met Val Ser Leu
           355                 360                 365
   Leu Pro Ala Ile Ile Glu Asn Leu Arg Asp Tyr Gly Leu Ser Pro Thr
       370                 375                 380
   Ala Ala Asp Lys Thr Glu Phe Ile Asp Val Cys Pro Leu Asn Lys Ile
   385                 390                 395                 400
   Ser Phe Leu Lys Arg Thr Phe Glu Leu Thr Asp Ile Gly Trp Val Ser
                   405                 410                 415
   Lys Leu Asp Lys Ser Ser Ile Leu Arg Gln Leu Glu Trp Ser Lys Thr
               420                 425                 430
   Thr Ser Arg His Met Val Ile Glu Glu Thr Tyr Asp Leu Ala Lys Glu
                   435                 440                 445
   Glu Arg Gly Val Gln Leu Glu Glu Leu Gln Val Ala Ala Ala Ala His
       450                 455                 460
   Gly Gln Glu Phe Phe Asn Phe Val Cys Arg Glu Leu Glu Arg Gln Gln
   465                 470                 475                 480
   Ala Tyr Thr Gln Phe Ser Val Tyr Ser Tyr Asp Ala Ala Arg Lys Ile
                   485                 490                 495
   Leu Ala Asp Arg Lys Arg Val Val Ser Val Val Pro Asp Asp Glu Phe
               500                 505                 510
   Val Asn Val Met Glu His His His His His His
           515                 520
<210> 16
   <211> 22
   <212> RNA
   <213> Homo sapiens
<400> 16
   uuuguucguu cggcucgcgu ga 22
<210> 17
   <211> 17
   <212> DNA
   <213> Artificial
<220>
   <223> Oligonucleotide DS16
<400> 17
   cgaacgaaca aaccccc 17
<210> 18
   <211> 10
   <212> DNA
   <213> Artificial
<220>
   <223> Oligonucleotide DS10
<400> 18
   tcacgcgagc 10

## Description

The present invention relates to a method for adding at least one ribonucleotide to the 3'-terminal deoxyribonucleotide of certain polynucleotides such as double-stranded DNA or DNA/RNA hybrid, the latter being either single- or double-stranded.

Terminal deoxynucleotidyl transferase is known to add deoxyribonucleotides to a 3' end of DNA.

WO-A-2007/012329 describes certain RNA-dependent RNA polymerases (RdRps), in particular RdRps of caliciviruses, having a terminal transferase activity on RNA.

The technical problem underlying the present invention is to provide a novel system for labelling DNA-containing polynucleotides.

The solution to the above technical problem is provided by the embodiments of the present invention as defined herein and in the claims.

According to the invention, it has surprisingly found that RdRps described in WO-A-2007/012329 have also a terminal transferase activity on DNA, although these enzymes display an RNA-dependent RNA polymerase activity *in vitro* (Rohayem et al. (2005), J. Virol. 79(8): 4977-90)

Thus, the present invention provides a method for adding at least one ribonucleotide to an unpaired 3'-terminal deoxyribonucleotide of a polynucleotide wherein said polynucleotide is selected from the group consisting of double-stranded polydeoxyribonucleotides having at least one 3' overhang and double-stranded polynucleotides containing deoxyribonucleotides and ribonucleotides and having at least one 3' overhang, comprising the step of incubating under terminal transferase conditions in the presence of the desired ribonucleotide the polynucleotide with an enzyme having a "right hand conformation" and having a primary sequence comprising a conserved arrangement of the following sequence motives:
a. XXDYS (SEQ ID NO: 1)
b. GXPSG (SEQ ID NO: 2)
c. YGDD (SEQ ID NO: 3)
d. XXYGL (SEQ ID NO: 4)
e. XXXXFLXRXX (SEQ ID NO: 5)
with the following meanings:
D: aspartate
Y: tyrosine
S: serine
G: glycine
P: proline
L: leucine
F: phenylalanine
R: arginine
X: any amino acid;

The so-called "right hand conformation" as used herein means that the tertiary structure (conformation) of the protein folds like a right hand with finger, palm and thumb, as observed in certain RNA-dependent RNA polymerases (RdRps).

The sequence motif "XXDYS" is the so-called A-motif. The A-motif is responsible for the discrimination between ribonucleosides and deoxyribonucleosides. The motif "GXPSG" is the so-called B-motif. The B-motif is conserved within all representatives of the RdRp family of the corresponding polymerases from *Calicivirdae.* The motif "YGDD" ("C-motif") represents the active site of the enzyme. This motif, in particular the first aspartate residue (in bold, YGDD) plays an important role in the coordination of the metal ions during the Mg²⁺/Mn²⁺-dependent catalysis. The motif "XXYGL" is the so-called D-motif. The D-motif is a feature of template-dependent polymerases. Finally, the "XXXXFLXRXX" motif ("E-motif") is a feature of RNA-dependent RNA polymerases which discriminates them from DNA-dependent RNA polymerases.

Typical representatives of the above types of RdRps are the corresponding enzymes of the calicivirus family (*Caliciviridae*). Preferred embodiments of the RdRp are corresponding enzymes of a human and/or non-human pathogenic calicivirus. Especially preferred is an RdRp of a norovirus, sapovirus, vesivirus or lagovirus, for example the RdRp of the norovirus strain HuCV/NL/Dresden174/1997/GE (GenBank Acc. No. AY741811) or of the sapovirus strain pJG-Sap01 (GenBank Acc. No. AY694184) or an RdRp of the vesivirus strain FCV/Dresden/2006/GE (GenBank Acc. No. DQ424892).

According to especially preferred embodiments of the invention the RdRp is a protein having an amino acid sequence according to SEQ ID NO: 6 (norovirus-RdRp), SEQ ID NO: 7 (sapovirus-RdRp), SEQ ID NO: 8 (vesivirus-RdRp) or SEQ ID NO: 9 (lagovirus-RdRp). The person skilled in the art is readily capable of preparing such RdRp, for example by recombinant expression using suitable expression vectors and host organisms (cf. WO-A-2007/012329). To facilitate purification of the RdRp in recombinant expression, it is preferred that the RdRp is expressed with a suitable "tag" (for example GST or (His)₆-tag) at the N- or C-terminus of the corresponding sequence. For example, a histidine tag allows the purification of the protein by affinity chromatography over a nickel or cobalt column in a known fashion. Examples of embodiments of RNA polymerases fused to a histidine tag are the proteins comprising (or having) an amino acid sequence according to SEQ ID NO: 10, SEQ ID NO: 11, SEQ ID NO: 12, SEQ ID NO: 13, SEQ ID NO: 14 or SEQ ID NO: 15. SEQ NO: NO: 10 and SEQ ID NO: 11 correspond to an RNA polymerase of a norovirus having a histidine tag (SEQ ID NO: 10: C-terminal His-tag; SEQ ID NO: 11: N-terminal His-tag). SEQ ID NO: 12 and SEQ ID NO: 13 correspond to amino acid sequences of an RNA polymerase of a sapovirus having a histidine tag (SEQ ID NO: 12: C-terminal His-tag; SEQ ID NO: 13: N-terminal His-tag). SEQ ID NO: 14 corresponds to the amino acid sequence of an RNA polymerase of a vesirius having a histidine tag (C-terminal). SEQ ID NO: 15 corresponds to the amino acid sequence of an RNA polymerase of a lagovirus having a histidine tag (C-terminal).

The ribonucleotide to be transferred to the 3' end of the polynucleotide may bear at least one chemical modification and/or a label. The chemical modification may be at the ribose or deoxyribose, base and/or phosphate moiety.

Preferred examples of ribose-modified ribonucleotides are analogues wherein the 2'-OH group is replaced by a group selected from H, OR, R, halo, SH, SR, NH₂, NHR, NR₂ or CN with R being C₁-C₆ alkyl, alkenyl or alkynyl and halo being F, Cl, Br or I.

Typical examples of such ribonucleotide analogues with a modified ribose at the 2' position include 2'-O-methyl-cytidine, 2'-amino-2'-deoxy-uridine, 2'-azido-2'-deoxy-uridine, 2'-fluoro-2'-deoxy-guanosine and 2'-O-methyl-5-methyl-uridine.

Examples of nucleotides leading to a phosphate backbone modification are phosphothioate analogues.

According to the present invention, the modified ribonucleotide may also be selected from analogues having a chemical modification at the base moiety. Examples of such analogues include 5-aminoallyl-uridine, 6-aza-uridine, 8-aza-adenosine, 5-bromo-uridine, 7-deaza-adenosine, 7-deaza-guanosine, N⁶-methyl-adenosine, 5-methyl- cytidine, pseudo-uridine, and 4-thio-uridine.

The method according to the invention is particularly useful for introducing a labelled ribonucleotide to the 3' end(s) of the polynucleotide that carries a label. The term "label" as used herein refers to any atom or molecule which can be used to provide a detectable (preferably quantifiable) signal, and which can be attached to a nucleic acid, in particular the oligonucleotide. Labels may provide signals detectable by fluorescence, radioactivity, colorimetry, gravimetry, X-ray diffraction or absorption, magnetism, enzymatic activity, and the like. Especially preferred labels according to the present invention are chromophores such as fluorescent labels

A variety of labels that would be appropriate for use in the present invention, as well as methods for their inclusion in the ribonucleotide, are known in the art and include, but are not limited to, enzymes (for example alkaline phosphatase and horseradish peroxidase) and enzyme substrates, radioactive atoms, fluorescent dyes, chromophores, chemiluminescent labels, electrochemiluminescent labels, such as Origen^{™} (Igen), ligands having specific binding partners or any other labels that may interact with each other to enhance, alter, or diminish a signal.

Among radioactive atoms, ³²P is preferred. Methods for introducing ³²P into nucleic acids are known in the art, and include, for example 5'-labeling with a kinase, or random insertion by nick translation. Enzymes are typically detected by their activity. "Specific binding partner" refers to a protein capable of binding a ligand molecule with high specificity, as for example in the case of an antigen and a monoclonal antibody specific therefore. Other specific binding partners include biotin and avidin or streptavidin, IgG and protein A, and the numerous receptor-ligand couples known in the art. The above description is not meant to categorize the various labels into distinct classes, as the same label may serve in several different modes. For example ¹²⁵I may serve as a radioactive label or as an electron-dense reagent. HRP may serve as enzyme or as antigen for a monoclonal antibody. Further, one may combine various labels for a desired effect. For example, one might label a probe with biotin, and detect the presence of the oligonucleotide with avidin labeled with ¹²⁵I, or with an anti-biotin monoclonal antibody labeled with HRP. Other permutations and possibilities will be readily apparent for the skilled person and considered as equivalents within the scope of the present invention.

Fluorophores for use as labels in constructing labeled polynucleotides of use according to the present invention are preferred and include rhodamine and derivatives, such as Texas Red, 5-carboxytetramethyl rhodamine (5-TAMRA), 6-carboxytetramethyl rhodamine (6-TAMRA) and their respective succinimidyl esters, fluorescein and derivatives, such as 5-bromomethyl fluorescein, 5-carboxy fluorescein (5-FAM), 6-carboxy fluorescein (6-FAM), and their respective succinimidyl esters, Lucifer Yellow, IAEDANS, 7-Me₂-N-coumarin-4-acetate, 7-OH-4-CH₃-coumarin-3-acetate, 7-NH₂-4-CH₃-coumarin-3-acetate (AMCA), monobromobimane, pyrene trisulfonates, such as Cascade Blue, and monobromotrimethyl-ammoniobimane.

The polynucleotide to be provided with at least one ribonucleotide at its 3'-end may be of any origin and length. The polynucleotide may be pure DNA such as a double-stranded DNA-molecule having one or two 3'-overhangs. It is to be understood that the polynucleotide according to the invention may also contain ribonucleotides, i.e. it may contain regions of RNA. The polynucleotide is double-stranded. In fact the polynucleotide may also be pure RNA except for the last 3'-terminal nucleotide which is, according to the present invention, a deoxyribonucleotide.

According to the invention it is clear that the 3'-terminal deoxyribonucleotide may also be part of, for example, a DNA molecule (e.g. a DNA oligonucleotide) hybridised to another polynucleotide species (e.g. an RNA) with the proviso that the 3'-terminal deoxyribonucleotide of the DNA (or DNA containing molecule) is unpaired, i.e. forms a free 3'-terminus.

According to the present invention, the term "terminal transferase conditions" means the conditions, in particular relating to buffer, temperature, salt and metal ion (if applicable), that allow the RdRp to add a ribonucleotide (or a ribonucleotide analogue or labelled ribonucleotide, as outlined above) to an unpaired 3'-terminal deoxyribonucleotide of a (partially) double-stranded polynucleotide or of a single-stranded polynucleotide. Appropriate buffer, salt, metal ion, reducing agent (if applicable) and other conditions of RdRps are known to the skilled person. With regard to RdRps of caliciviruses, it is referred to WO-A-2007/012329. Thus, in typical examples of the methods according to the present invention, the concentration of the desired ribonucleoside triphosphate (rCTP or rGTP or rATP or rUTP or a chemically modified or labelled analogue thereof) is preferably in the range of from 0.1 mM to 1 mM, for example 0.4 µmol/l. The concentration of the RdRp may be for example 1 µM to 6 µM.

Typical buffer conditions are 10 to 80 mM, more preferred 20 to 50 mM HEPES pH 8.0, 1 to 200 mM, for example 5 to 150 mM, particularly preferred 80 to 120 mM, most preferably 100 mM magnesium acetate, magnesium chloride, manganese acetate or manganese chloride and 1 to 4 mM of a reducing agent, for example DTT. With respect to the terminal transferase activity of preferred enzymes according to the present invention it is further referred to Rohayem et al. (2006) Journal of General Virology 87, 2621-2630, and the reaction conditions for terminal transferase activity of calicivirus RdRps (in that case on RNA species though) described therein.

The method according to the present invention may be stopped by introducing a stop solution into the reaction mixture. A typical stop solution contains 2 to 10 mM, preferably 4 to 8 mM ammonium acetate and 50 to 200 mM, for example 100 mM EDTA.

For practicing the methods of the present invention, a thermal cycler, such as the commercially available machines from Perkin-Elmer Instruments or Roche Diagnostics may be employed.

The present invention is further illustrated by the accompanied drawings showing results of exemplary embodiments of the invention:

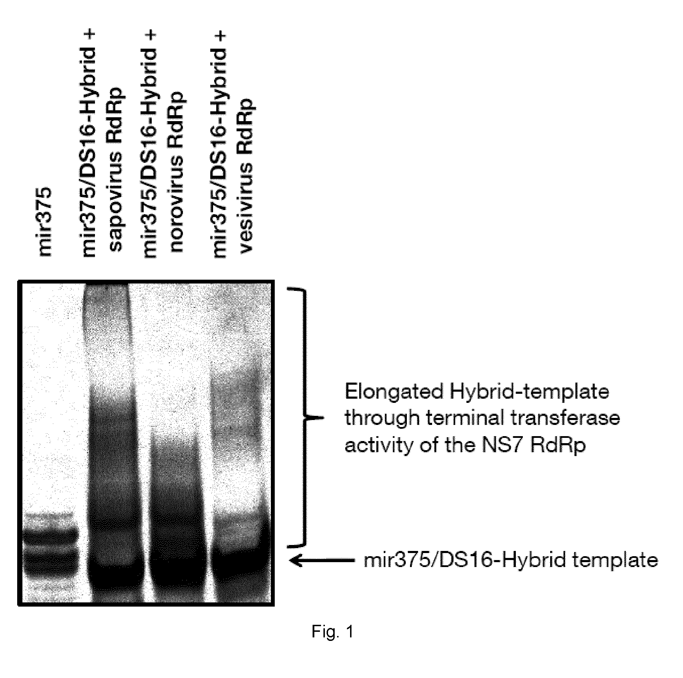

- Fig. 1:: Terminal Transferase activity of a sapovirus, norovirus and vesivirus RdRp on the DNA/RNA hybrid template mir375/DS16. One microgramm of miR375-RNA (5'-UUUGUUCGUUCGGCUCGCGUGA-3'; SEQ ID NO: 16) was incubated with 1 µl of buffer A (50 mM, Tri-Hcl, 100 mM NaCl, 5 mM EDTA, pH 8.0) and 3 µl of RNase-DNase-free water at 95 °C for 5 min. In the next step, the oligonucleotide DS16 (5'-CGAACGAACAAACCCCC-3'; SEQ ID NO: 17) labeled at its 5' end with TAMRA was added to the reaction (final volume 5µl) at a final concentration of 15 µM together with 1 µl buffer A. The mix was incubated at 65 °C for 30 min followed by cooling on ice for 15 min. Hybridisation of DS16 to mir375 yielded the mir375/DS16 hybrid. The following reagents were then added to the reaction: 4 µl buffer B (Hepes 250 mM, MnCl2 25 mM, DTT 5 mM, pH 7.6), 2 mM CTP, 8 µM of sapovirus RRp, norovirus RdRp or vesivirus RdRp, and RNase-DNase-free water to a total volume of 20 µl. The reaction mix was incubated at 30 °C for 30 min. The reaction was then loaded on a 20% native polyacrymalide gel and the terminal transferase activity evidenced by electrophoresis after ethidium bromide staining and UV transillumination. The product of the terminal transferase activity of the RdRps on the mir375/DS16 hybrid template in the presence of rCTP is indicated.

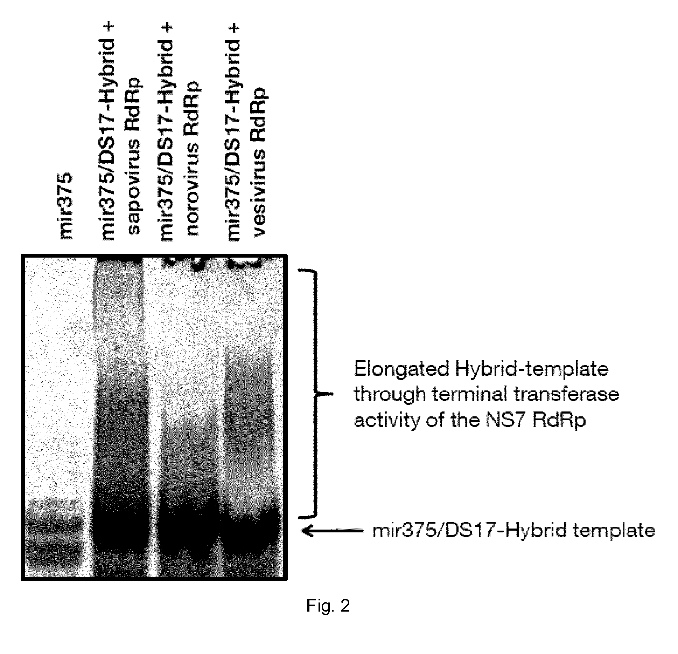

- Fig. 2: Terminal transferase activity of a sapovirus, norovirus and vesivirus RdRp on the DNA/RNA hybrid template mir375/DS17. One microgramm of miR375-RNA (5'-UUUGUUCGUUCGGCUCGCGUGA-3'; SEQ ID NO: 16) was incubated with 1 µl of buffer A (50 mM, Tri-Hcl, 100 mM NaCl, 5 mM EDTA, pH 8.0) and 3 µl of RNase-DNase-free water at 95 °C for 5 min. In the next step, the oligonucleotide DS17 (5'-CGAACGAACAAACCCCCCCCCC-3'; SEQ ID NO: 18) labeled at its 5' end with TAMRA was added to the reaction (final volume 5µl) at a final concentration of 15 µM together with 1 µl buffer A. The mix was incubated at 65 °C for 30 min followed by cooling on ice for 15 min. Hybridisation of DS16 to mir375 yielded the mir375/DS17 hybrid. The following reagents were then added to the reaction: 4 µl buffer B (Hepes 250 mM, MnCl2 25 mM, DTT 5 mM, pH 7.6), 2 mM CTP, 8 µM of sapovirus RdRp, norovirus RdRp or vesivirus RdRp, and RNase-DNase-free water to a total volume of 20 µl. The reaction mix was incubated at 30 °C for 30 min. The reaction was then loaded on a 20% native polyacrymalide gel and the terminal transferase activity evidenced by electrophoresis after ethidium bromide staining and UV transillumination. The product of the terminal transferase activity of the RdRps on the mir375/DS17 hybrid template in the presence of rCTP is indicated.

## Claims

1. A method for adding at least one ribonucleotide to an unpaired 3'-terminal deoxyribonucleotide of a polynucleotide wherein said polynucleotide is selected from the group consisting of double stranded polydeoxyribonucleotides having at least one 3' overhang and double stranded polynucleotides containing deoxyribonucleotides and ribonucleotides and having at least one 3' overhang comprising the step of incubating under terminal transferase conditions in the presence of the desired ribonucleotide the polynucleotide with an enzyme having a "right hand conformation" and having a primary sequence comprising a conserved arrangement of the following sequence motives:
a. XXDYS (SEQ ID NO: 1)
b. GXPSG (SEQ ID NO: 2)
c. YGDD (SEQ ID NO: 3)
d. XXYGL (SEQ ID NO: 4)
e. XXXXFLXRXX (SEQ ID NO: 5)
with the following meanings:
D: aspartate
Y: tyrosine
S: serine
G: glycine
P: proline
L: leucine
F: phenylalanine
R: arginine
X: any amino acid.

2. The method of claim 1 wherein the enzyme is an RNA-dependent RNA polymerase (RdRp) of a virus of the *Caliciviridae* family.

3. The method of claim 2 wherein the RdRp is selected from the group consisting of RdRps of a norovirus, sapovirus, vesivirus and lagovirus.

4. The method of claim 3 wherein the RdRp is selected from the group consisting of an RdRp of the norovirus strain HuCV/NL/Dresden174/1997/GE (GenBank Acc. No. AY741811), an RdRp the sapovirus strain pJG-Sap01 (GenBank Acc. No. AY694184) and an RdRp of the vesivirus strain FCV/Dresden/2006/GE (GenBank Acc. No. DQ424892).

5. The method of claim 3 wherein the RdRp is a protein having an amino acid sequence selected from the group consisting of SEQ ID NO: 6 to 15.

6. The method according to any one of the preceding claims wherein the at least one ribonucleotide to be added is chemically modified and/or labelled.

7. The method of claim 6 wherein the ribonucleotide to be added is chemically modified at the ribose, phosphate and/or base moiety.

8. The method of claim 6 wherein the label is selected from the group consisting of radioactive atoms, fluorescent dyes, chromophores, chemiluminescent labels, electrochemiluminescent labels and ligands having specific binding partners.

9. The method according to any one of the preceding clams wherein the polynucleotide is DNA.

10. The method according to any one of the preceding claims wherein the polynucleotide is an RNA/DNA hybrid.

## Patentansprüche

1. Verfahren zur Anfügung von mindestens einem Ribonukleotid an ein nichtgepaartes 3'-terminales Desoxyribonukleotid eines Polynukleotids, wobei das Polynukleotid aus der Gruppe, bestehend aus doppelsträngigen Polydesoxyribonukleotiden mit mindestens einem 3'-Überhang und doppelsträngigen Polynukleotiden, die Desoxyribonukleotide und Ribonukleotide enthalten und mindestens einen 3'-Überhang aufweisen, ausgewählt ist, umfassend den Schritt des Inkubierens des Polynukleotids unter Terminale-Transferase-Bedingungen in Gegenwart des gewünschten Ribonukleotids mit einem Enzym, das eine "Rechte-Hand-Konformation" und eine Primärsequenz aufweist, die eine konservierte Anordnung der folgenden Sequenzmotive umfasst:
a. XXDYS (SEQ ID NO: 1)
b. GXPSG (SEQ ID NO: 2)
c. YGDD (SEQ ID NO: 3)
d. XXYGL (SEQ ID NO: 4)
e. XXXXFLXRXX (SEQ ID NO: 5)
mit den folgenden Bedeutungen:
D: Asparat
Y: Tyrosin
S: Serin
G: Glycin
P: Prolin
L: Leucin
F: Phenylalanin
R: Arginin
X: irgendeine Aminosäure

2. Verfahren nach Anspruch 1, wobei das Enzym eine RNA-abhängige RNA-Polymerase (RdRp) eines Virus der Familie der *Caliciviridae* ist.

3. Verfahren nach Anspruch 2, wobei die RdRp aus der Gruppe, bestehend aus RdRps eines Norovirus, Sapovirus, Besivirus und Lagovirus, ausgewählt ist.

4. Verfahren nach Anspruch 3, wobei die RdRp aus der Gruppe, bestehend aus einer RdRp des Norovirus-Stammes HuCV/NL/Dresden174/1997/GE (GenBank Zugriffsnr. AY741811), einer RdRp des Sapovirus-Stammes pJG-Sap01 (GenBank Zugriffsnr. AY694184) und einer RdRp des Vesivirus-Stammes FCV/Dresden/2006/GE (GenBank Zugriffsnr. DQ424892), ausgewählt ist.

5. Verfahren nach Anspruch 3, wobei die RdRp ein Protein ist, das eine Aminosäuresequenz aufweist, die aus der Gruppe, bestehend aus SEQ ID NO: 6 bis 15, ausgewählt ist.

6. Verfahren nach einem der vorhergehenden Ansprüche, wobei das mindestens eine anzufügende Ribonukleotid chemisch modifiziert und/oder markiert ist.

7. Verfahren nach Anspruch 6, wobei das anzufügende Ribonukleotid an der Ribose, dem Phosphat und/oder der Baseneinheit chemisch modifiziert ist.

8. Verfahren nach Anspruch 6, wobei die Markierung aus der Gruppe, bestehend aus radioaktiven Atomen, fluoreszierenden Farbstoffen, Chromophoren, chemilumineszierenden Markierungen, elektrolumineszierenden Markierungen und Liganden mit spezifischen Bindungspartnern, ausgewählt ist.

9. Verfahren nach einem der vorhergehenden Ansprüche, wobei das Polynukleotid DNA ist.

10. Verfahren nach einem der vorhergehenden Ansprüche, wobei das Polynukleotid ein RNA/DNA-Hybrid ist.

## Revendications

1. Procédé pour ajouter au moins un ribonucléotide à l'extrémité 3' terminale non appariée d'un désoxyribonucléotide d'un polynucléotide dans lequel le polynucléotide est choisi dans le groupe constitué des polydésoxyribonucléotides double brin ayant au moins une extrémité 3' cohésive et les polynucléotides doubles brin contenant des désoxyribonucléotides et ribonucléotides et présentant au moins une extrémité cohésive 3', comprenant une étape d'incubation du polynucléotide dans les conditions d'une transférase terminale, en présence du ribonucléotide souhaité, avec une enzyme ayant une "conformation main droite" et dont la séquence primaire comprend un arrangement conservé des motifs de séquence suivants:
a. XXDYS (SEQ ID NO: 1)
b. GXPSG (SEQ ID NO: 2)
c. YGDD (SEQ ID NO: 3)
d. XXYGL (SEQ ID NO: 4)
e. XXXXFLXRXX (SEQ ID NO: 5)
avec les significations suivantes:
D: aspartate
Y: tyrosine
G: glycine
P: proline
L: leucine
F: phenylalanine
R: arginine
X: n'importe quel acide aminé.

2. Procédé selon la revendication 1, selon lequel l'enzyme est une ARN polymérase ARN-dépendante (RdRp) d'un virus de la famille des Caliciviridae.

3. Procédé selon la revendication 2, selon lequel la RdRp est choisie dans le groupe constitué des RdRps de norovirus, sapovirus, vesivirus et lagovirus.

4. Procédé selon la revendication 3, selon lequel la RdRp est choisie dans le groupe constitué d'une RdRp de la souche de norovirus HuCV/NL/Dresden174/1997/GE (GenBank Acc. No AY741811), une RdRp de la souche sapovirus pJG-Sap01 (GenBank Ace. No AY694184) et une RdRp de la souche du Vesivirus souche FCV/Dresde/2006/GE (GenBank Ace. No. DQ424892).

5. Procédé de la revendication 3, selon lequel la RdRp est une protéine ayant une séquence d'acides aminés choisie dans le groupe consititué des SEQ ID NO: 6 à 15.

6. Procédé selon l'une quelconque des revendications précédentes, selon lequel le au moins un ribonucléotide devant être ajouté est chimiquement modifié et / ou marqué.

7. Procédé selon la revendication 6, selon lequel le ribonucléotide devant être ajouté est modifié chimiquement au niveau d'une entité ribose, phosphate et / ou base.

8. Procédé selon la revendication 6, selon lequel le marqueur est choisi dans le groupe constitué par les atomes radioactifs, les colorants fluorescents, les chromophores, les marqueurs chimioluminescents, les marqueurs électrochimioluminescents et les ligands ayant des partenaires de liaison spécifiques.

9. Procédé selon l'une quelconque des revendications précédentes, selon lequel le polynucléotide est de l'ADN double brin.

10. Procédé selon l'une quelconque des revendications précédentes, selon lequel le polynucléotide est un hybride ARN / ADN.
